# EUROPEAN PATENT APPLICATION

(11) **EP 4 512 423 A1**
(43) Date of publication of application: **26.02.2025**
(21) Application number: 23791829.7
(22) Date of filing: 17.04.2023
(51) Int. Cl.: A61K 45/00, A61K 39/395, A61P 35/02, C07K 16/28, C12N 15/13

(54) **THERAPEUTIC AGENT FOR NK CELL TUMOR**

(30) Priority: 19.04.2022 JP 2022068757
(71) Applicant: Tokai University Educational System, Tokyo, 151-0063 (JP); Perseus Proteomics Inc., Tokyo 103-0015 (JP)
(72) Inventor: YOSHIDA KOTANI Ai, Isehara-shi, Kanagawa 259-1193 (JP); UKAI Yoshinori, Tokyo 1030015 (JP); MATSUURA Tadashi, Tokyo 1030015 (JP); ISHII Keisuke, Tokyo 1030015 (JP)
(74) Representative: Hoffmann Eitle
(86) International application number: PCT/JP2023/015337
(87) International publication number: WO 2023/204181

(57) **Abstract**

It is an object of the present invention to provide a therapeutic agent for NK-cell tumor. According to the present invention, a therapeutic agent for NK-cell tumor, which comprises a substance that recognizes a transferrin receptor, is provided.

## Description

### Technical Field

The present invention relates to a therapeutic agent for NK-cell tumor, which comprises a substance that recognizes a transferrin receptor.

### Background Art

Transferrin receptor (TfR) has been first identified to be a receptor that is present on a reticulocyte as a cell membrane structure for incorporating transferrin (Tf)-bound iron into a cell. Thereafter, it is discovered that TfR is expressed in placental trophoblasts, in activated lymphocytes, and further, in various tumor cells, etc. For example, the high expression of TfR in breast cancer, prostate cancer, lung cancer, pancreatic cancer, colon cancer, stomach cancer, bladder cancer, hepatic cancer, cervical cancer, brain tumor, chronic lymphocytic leukemia, non-Hodgkin's lymphoma, and adult T-cell leukemia has been reported. Moreover, since TfR is expressed on the surface of various types of cancer cells at a high level and is expressed in normal cells at a low level, this receptor is recognized as a molecular target for cancer therapy. For example, Patent Document 1 discloses an antibody capable of specifically recognizing the transferrin receptor and an anti-cancer agent using the aforementioned antibody.

On the other hand, examples of the known NK-cell tumor may include three disease types, namely, aggressive NK-cell leukemia (ANKL), extranodal NK/T-cell lymphoma, nasal type (ENKL), and chronic lymphoproliferative disorders of NK cells (CLPD-NK). Aggressive NK-cell leukemia is a malignant lymphoma (blood cancer) derived from NK (natural killer) cells as one type of immune cells. Aggressive NK-cell leukemia is a fulminant, refractory malignant disease of the hematopoietic system that rapidly worsens after the onset. The incidence of such aggressive NK-cell leukemia is restricted to only a few regions in Asia and Latin America, and the number of new cases in Japan is only a few dozen per year. Thus, since the aggressive NK-cell leukemia is an extremely rare disease, the onset cause of the disease has not been elucidated, and it has been desired to establish an effective standard treatment method for this disease as soon as possible.

### Prior Art Document

### Patent Document

Patent Document 1: International Publication WO2014/073641

### Summary of Invention

### Object to be Solved by the Invention

It has been known that a substance that recognizes a transferrin receptor is used as an anticancer agent. However, whether or not such transferrin receptor-recognizing substance exhibits therapeutic effects on NK-cell tumor been unknown. It is an object of the present invention to provide a therapeutic agent for NK-cell tumor.

### Means for Solving the Object

As a result of intensive studies conducted directed towards achieving the above-described object, the present inventors have found that a transferrin receptor-binding substance exhibits therapeutic effects on NK-cell tumor, thereby completing the present invention.

Specifically, according to the present invention, the following inventions are provided.
(1) A therapeutic agent for NK-cell tumor, which comprises a substance that recognizes a transferrin receptor.
(2) The therapeutic agent according to (1), wherein the NK-cell tumor is aggressive NK-cell leukemia, or extranodal NK/T-cell lymphoma, nasal type (ENKL).
(3) The therapeutic agent according to (1) or (2), wherein the transferrin receptor-recognizing substance is a transferrin receptor-recognizing antibody.
(4) The therapeutic agent according to (3), wherein the transferrin receptor-recognizing antibody is a human transferrin receptor-recognizing antibody.
(5) The therapeutic agent according to (4), wherein the human transferrin receptor-recognizing antibody is an antibody that recognizes the amino acids at positions 629 to 633 of a human transferrin receptor, or an antibody that inhibits the binding of another antibody to the amino acids at positions 629 to 633 of a human transferrin receptor.
(6) The therapeutic agent according to any one of (3) to (5), wherein the antibody is an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and also having a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.
(7) The therapeutic agent according to any one of (3) to (6), wherein the antibody is an antibody having a heavy chain as set forth in SEQ ID NO: 7 and a light chain as set forth in SEQ ID NO: 8.
(8) The therapeutic agent according to any one of (3) to (7), wherein the antibody is a human antibody or a humanized antibody.
(9) The therapeutic agent according to any one of (3) to (8), wherein the antibody is an antibody fragment selected from the group consisting of Fab, Fab', F(ab')₂, a single-chain antibody (scFv), a multi-specific antibody, a disulfide-stabilized V region (dsFv) and a peptide comprising CDR.

(A) A method for treaing NK-cell tumor, which comprises administering a substance that recognizes a transferrin receptor to a subject.
(B) A substance that recognizes a transferrin receptor for use in treatmemt of NK-cell tumor.
(C) Use of a substance that recognizes a transferrin receptor for production of a therapeutic agent for NK-cell tumor.

### Advantageous Effects of Invention

The therapeutic agent of the present invention is useful for the treatment of NK-cell tumor.

### Brief Description of Drawings

[Fig. 1] Fig. 1 shows the sites of individual TfR mutant fragments, at which point mutations are performed.
[Fig. 2] Fig. 2 shows the reactivity of TfR436 with soluble wild-type TfR (sTfR) and TfR mutant fragments.
[Fig. 3] Fig. 3 shows the Tf-TfR binding inhibitory activity of TfR436.
[Fig. 4] Fig. 4 shows the results of a test regarding the cytotoxicity of TfR436 against cell lines derived from NK-cell tumor.
[Fig. 5] Fig. 5 shows the inhibitory effect of TfR436 on ANKL cells in mice.
[Fig. 6] Fig. 6 shows the inhibitory effect of TfR436 on ANKL cells in mice.
[Fig. 7] Fig. 7 shows a transition in the body weight of mice.
[Fig. 8] Fig. 8 shows the results of the survival test of mice.
[Fig. 9] Fig. 9 shows an *in vivo* competitive assay method using a CRISPR-Cas9 system.
[Fig. 10] Fig. 10 shows a comparison of sgNT- or sgTFRC-introduced ANKL1-Cas9 cells in the liver.
[Fig. 11] Fig. 11 shows the viability of ANKL cell lines treated with various concentrations of TfR436 antibody.
[Fig. 12] Fig. 12 shows the viability of primary ANKL cells treated with various concentrations of TfR436 antibody.
[Fig. 13] Fig. 13 shows the procedures for validating the efficacy of TfR436 antibody *in vivo* using ANKL-PDX mice.
[Fig. 14] Fig. 14 shows the ratio of human CD45+ cells to mouse CD45+ cells.
[Fig. 15] Fig. 15 shows the method of RNAseq analysis of ANKL1-PDX mice.
[Fig. 16] Fig. 16 shows a volcano plot of RNA-seq data.
[Fig. 17] Fig. 17 shows the results of the measurement of iron amount in ANKL3 cells.
[Fig. 18] Fig. 18 shows the procedures for *in vivo* evaluation of the therapeutic effects of a TfR436 antibody.
[Fig. 19] Fig. 19 shows the survival curves of ANKL1, ANKL3 and ANKL5 PDX mice.
[Fig. 20] Fig. 20 shows the procedures for evaluating the efficacy of a treatment with a TfR436 antibody in ANKL-PDX mice with advanced symptoms.
[Fig. 21] Fig. 21 shows the therapeutic effects of a TfR436 antibody in ANKL1-PDX mice with advanced symptoms.
[Fig. 22] Fig. 22 shows the therapeutic effects of a TfR436 antibody in ANKL1-PDX mice with advanced symptoms.
[Fig. 23] Fig. 23 shows the IVIS analysis of ANKL1-PDX mice treated with a TfR436 antibody or PBS.
[Fig. 24] Fig. 24 shows the results of immunostaining of livers from mice administered with a TfR436 antibody.

### Embodiment of Carrying out the Invention

Hereinafter, the present invention will be described in more detail.

### Definitions and General Techniques

Unless otherwise specified in the present description, scientific terms used regarding the present invention include meanings that are generally understood by a person skilled in the art. In general, nomenclatures and techniques applied to the cell and tissue culture, molecular biology, immunology, microbiology, genetics, protein and nucleic acid chemistry, and hybridization, which are described in the present description, are well known in the present technical field, and thus, are commonly used.

The methods and techniques of the present invention are carried out in accordance with conventional methods that are well known in the present technical field, in such ways as described in a variety of general reference documents cited and discussed throughout the present description and more specific reference documents, unless otherwise specified.

### TfR

In the case of a human, transferrin receptor (TfR) is a single-pass transmembrane protein (SEQ ID NO: 9) composed of 760 amino acids, and it is encoded by human chromosome 3. This protein has also been known as a CD71 antigen, and it is considered that this protein is associated with incorporation of iron into cells and cell growth. The structure of the TfR of the present invention is not particularly limited, and thus, the TfR of the present invention includes all of a monomer, a polymer, an intact form expressed on a cell membrane, a soluble form constituted in an extracellular region, a truncated form, a mutation form caused by genetic mutation, deletion, etc., and a form that has undergone posttranslational modification by phosphorylation, and the like.

### React and Reactivity

The terms "react" and "reactivity" have the same meanings in the present description, unless otherwise specified. That is, these terms mean that an antibody recognizes an antigen. The antigen used herein may be any of an intact TfR expressed on a cell membrane, a truncated form, and a soluble form. In addition, the antigen may be either a TfR having a three-dimensional structure or a modified TfR. Examples of a means for examining reactivity include flow cytometry (FACS), enzyme-linked immunosorbent assay (ELISA), Western blotting, microfluorescence measuring technique (FMAT), surface plasmon resonance (Biacore), immunostaining, and immunoprecipitation.

The antibody used in flow cytometry may be either an antibody labeled with a fluorescent substance such as FITC or with biotin, or an unlabeled antibody. A fluorescently-labeled avidin, a fluorescently-labeled anti-human immunoglobulin antibody, or the like is used, depending on the presence or absence of labeling of the antibody used and the type thereof. Reactivity can be evaluated by adding a sufficient amount of anti-TfR antibody (generally having a final concentration of 0.01 to 10 µg/mL) to an analyte, and then by comparing the obtained reactivity with the reactivity with a negative control antibody or a positive control antibody.

### Substance that recognizes a transferrin receptor

In the present invention, a substance that recognizes a transferrin receptor is used. Such a substance that recognizes a transferrin receptor is not particularly limited. For example, at least one selected from the group consisting of nucleic acids (nucleic acid aptamers, decoy nucleic acids, etc.), ribozymes, antibodies and fragments thereof, peptides, cyclic peptides, and peptide mimics, can be used. For example, aptamers that mimic transferrin are described in Wilner et al., Molecular Therapy-Nucleic Acids (2012) 1, e21; doi:10.1038/mtna.2012.14. On the other hand, peptides that target transferrin receptors are described in Jae H. Lee et al., Eur. J. Biochem. 268, 2004-2012 (2001). In the present invention, such nucleic acids, peptides and the like can be used.

### Antibody

In the present description, the following abbreviations (in the parentheses) are used in accordance with the customs, as necessary.

Heavy chain (H chain), light chain (L chain), heavy chain variable region (VH), light chain variable region (VL), complementarity determining region (CDR), first complementarity determining region (CDR1), second complementarity determining region (CDR2), third complementarity determining region (CDR3), heavy chain first complementarity determining region (VH CDR1), heavy chain second complementarity determining region (VH CDR2), heavy chain third complementarity determining region (VH CDR3), light chain first complementarity determining region (VL CDR1), light chain second complementarity determining region (VL CDR2), and light chain third complementarity determining region (VL CDR3).

In the present description, the term "antibody" has the same definitions as immunoglobulin, and should be understood as generally known in the present technical field. Specifically, the term "antibody" is not limited by any given specific method for producing the antibody. For example, the term "antibody" includes, but is not limited to, a recombinant antibody, a monoclonal antibody, and a polyclonal antibody.

In the present description, the term "human antibody" is used to mean any given antibody, in which the sequences of a variable region and a constant region are human sequences. This term includes antibodies which have human sequences and are modified, for example, to remove cysteine that may cause a possible decrease in immunogenicity, an increase in affinity, and undesirable folding. This term also includes antibodies produced in non-human cells by recombination, which enable glycosylation that is not specific to human cells. These antibodies can be prepared in various ways.

In the present description, the term "humanized antibody" means a non-human-derived antibody, in which amino acid residues characteristic for a non-human antibody sequence are substituted with residues found in positions corresponding to those of a human antibody. This "humanization" process is considered to reduce the immunogenicity of the obtained antibody in human. It would be understood that a non-human-derived antibody can be humanized using a technique well known in the present technical field. Please refer to, for example, Winter et al., Immunol. Today 14: 43-46 (1993). The target antibody can be produced by an engineering approach via a recombination DNA technique of substituting CH1, CH2, CH3, a hinge domain, and/or a framework domain with those of the corresponding human sequence. For example, WO92/02190, and U. S. Patent Nos. 5,530,101, 5,585,089, 5,693,761, 5,693,792, 5,714,350 and 5,777,085 can be referred. In the present description, the term "humanized antibody" includes a chimeric human antibody and a CDR-grafted antibody, within the definitions thereof.

The sequence of a framework region (FR) in a variable region of the antibody is not particularly limited, unless it substantially affects the specific binding ability of the antibody to the corresponding antigen. The FR region of a human antibody is preferably used, but it is also possible to use FR regions of animal species other than humans (e.g. a mouse or a rat).

In one aspect, the antibody comprises a constant region as well as a variable region (e.g. IgG antibody). The sequence of such a constant region is not particularly limited. For example, the constant region of a known human antibody can be used. The heavy chain constant region (CH) of a human antibody is not particularly limited, as long as it belongs to a human immunoglobulin (hereinafter referred to as "hIg"). Those of hIgG class are preferable, and any one of subclasses belonging to hIgG class, such as hIgG1, hIgG2, hIgG3 or hIgG4, may be used. On the other hand, the light chain constant region (CL) of a human antibody is not particularly limited, as long as it belongs to hIg and those of κ class or λ class can be used. In addition, constant regions of animal species other than humans (e.g. a mouse or a rat) can also be used.

In the present description, the term "modified body" or "modified antibody" is used to mean that the amino acid sequence of the variable region (CDR sequences and/or FR sequences) of a parent antibody comprises a substitution, deletion, addition and/or insertion of one or multiple amino acids.

In the present description, the "parent antibody" means a TfR436 antibody that has a VH comprising the amino acid sequence as set forth in SEQ ID NO: 7 and a VL comprising the amino acid sequence as set forth in SEQ ID NO: 8. In the amino acid sequence, one or several (for example, 1 to 8, preferably 1 to 5, more preferably 1 to 3, and particularly preferably 1 or 2) amino acids are deleted, added, substituted and/or inserted. As a method of preparing the amino acid sequence of an antibody having a binding ability to TfR, which has been well known to a person skilled in the art, a method of introducing a mutation into a protein has been known. For instance, such a skilled person could prepare a modified antibody functionally equivalent to an antibody having a TfR-binding activity by appropriately introducing a mutation into the amino acid sequence of the antibody having a TfR-binding activity according to a site-directed mutagenesis (Hashimoto-Gotoh, T, Mizuno, T, Ogasahara, Y, an DNA Kagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci USA. 82, 488-492). Thus, an antibody comprising a mutation of one or several amino acids in the variable region or constant region of a parent antibody and having a binding activity to TfR can also be used.

In the present description, the phrase "an activity equivalent to the activity of the parent antibody" is used to mean that the binding activity of a certain antibody to human TfR is equivalent to that of the parent antibody thereof. The term "equivalent" does not necessarily mean the same level of activity. The activity may be increased, or the activity may also be decreased, as long as the antibody has the activity. An antibody having a decreased activity may be an antibody having an activity that is, for example, 30% or more, preferably 50% or more, more preferably 80% or more, further preferably 90% or more, and particularly preferably 95% or more of the activity of the original antibody.

The term "binding activity" means the activity of an antibody to recognize an antigen. This antigen may be an intact TfR expressed on a cell membrane, a truncated form, or a soluble form. In addition, the antigen may be either a TfR having a three-dimensional structure or a modified TfR. Examples of a means for examining the binding activity include flow cytometry (FACS), enzyme-linked immunosorbent assay (ELISA), Western blotting, microfluorescence measuring technique (FMAT), and surface plasmon resonance (Biacore).

The Tf-TfR binding inhibitory activity of an antibody can be measured according to the method described later in "Example 2(2) Comparison between TfR436 antibody and antibodies of other companies in terms of Tf-TfR binding inhibition." That is, a TfR solution is dispensed into a substrate (a 96-well plate, etc.) and is then left at rest to obtain a solid phase, and thereafter, blocking is carried out. Subsequently, an HRP-labeled Tf solution is dispensed into the substrate, and an antibody is further added thereto, so that they are allowed to react with each other at room temperature. Thereafter, the substrate is washed, and a coloring reagent (TMB, etc.) is then added thereto, so that they are allowed to react with each other. After that, the absorbance is measured using a plate reader. By performing the above-described operations, the Tf-TfR binding inhibitory activity of the antibody can be evaluated.

The antibody is not limited by its origin, and it may be an antibody derived from any animal, such as a human antibody, a mouse antibody, or a rat antibody. Also, the present antibody may be a chimeric antibody or a humanized antibody. One preferred aspect of the antibody of the present invention is a human antibody.

The antibodies may be different from one another in terms of amino acid sequence, molecular weight, isoelectric point, the presence or absence of a sugar chain or the form thereof, etc., depending on the after-mentioned cells or hosts that produce the antibodies, or a purification method. For example, an antibody that undergoes a posttranslational modification on the amino acid sequence described in the present description is included in the present invention. Moreover, an antibody that undergoes a posttranslational modification on a site other than those for the known posttranslational modification is also included in the present invention. Furthermore, when the antibody is allowed to express in prokaryotic cells such as *Escherichia coli,* a methionine residue is added to the N-terminus of the amino acid sequence of the original antibody. Such an antibody may also be used in the present invention. An antibody that undergoes a posttranslational modification on a site other than those for the known posttranslational modification is also included in the present invention.

### Production of Antibody

Either a monoclonal antibody or a polyclonal antibody may be used as an antibody in the present invention. Such a monoclonal antibody and a polyclonal antibody can be produced by methods known to those skilled in the art.

Examples of such antibodies may include antibodies produced in animal blood, antibodies produced by hybridomas, antibodies produced by a host that is transformed with an expression vector containing an antibody gene according to a genetic engineering method, antibodies obtained by screening optimal antibodies from a clone library according to phage display and then producing the genes of the antibodies in CHO cells, and antibodies directly obtained as human antibodies, using transgenic mice that produce such human antibodies.

In order to produce a polyclonal antibody, serum is obtained by immunizing an animal such as a rabbit with an antigen. The obtained serum is purified, for example, by ammonium sulfate precipitation, protein A column, protein G column, DEAE ion exchange chromatography, or affinity column, so as to prepare a polyclonal antibody.

In order to produce a monoclonal antibody, an animal is immunized with an antigen, together with an adjuvant, as desired. After confirming that a desired antibody level has increased in the serum of the immunized animal, immune cells (splenic cells, etc.) are harvested from the animal, and the harvested cells are then fused with mammalian myeloma cells. Hybridomas obtained by the cell fusion can be selected by culturing them in a normal selective culture medium, such as, for example, a HAT culture medium (i.e. a culture medium containing hypoxanthine, aminopterin and thymidine). Thereafter, a limiting dilution method is carried out, so that hybridomas producing antibodies of interest can be screened.

Production of an antibody according to a phage display method will be described below.

### (1) Obtaining of scFv reacting with antigen using phage display library

Using a phage display technique, a library comprising a repertoire of antibodies having various affinity for TfR can be provided. Subsequently, such a library can be screened to identify and isolate antibodies against TfR. Preferably, the phage library is a scFv phage display library that is generated using human VL and VH cDNA that has been prepared from mRNA isolated from human B cells. A method of preparing and screening such a library is known in the present technical field. A genetic substance is recovered from phage clones exhibiting reactivity that have been screened using TfR as an antigen. By analyzing the selected phage gene, the DNA sequences of VH and VL encoding the variable region of a human antibody binding to the antigen can be determined. Using this scFv sequence, IgG is prepared from scFv, so as to obtain a human antibody.

### (2) Preparation of IgG from scFv (preparation of human antibody)

An H chain or L chain expression vector is produced, and it is then allowed to express in a host cell. Thereafter, the secreted supernatant is recovered and is then purified, so as to obtain a human antibody. Alternatively, such a human antibody can also be obtained by allowing VH and VL to express in a single vector (tandem type). These methods are well known, and can be carried out with reference to WO92/01047, WO92/20791, WO93/06213, WO93/11236, WO93/19172, WO95/01438, WO95/15388, WO97/10354, etc.

Specifically, DNA encoding VH is ligated to another DNA molecule encoding a heavy chain constant region (CH1, CH2 and CH3), so as to obtain a full-length heavy chain gene. The sequence of a human heavy chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The heavy chain constant region may be the constant region of IgG1, IgG2, IgG3, IgG4, IgA, IgE, IgM or IgD. The most preferred constant region is that of IgG1 or IgG2. The constant region sequence of IgG1 may include any given various alleles or allotypes known to be generated among different individuals, such as Gm (1), Gm (2), Gm (3) or Gm (17). These allotypes correspond to a substitution of amino acids naturally-occurring in the constant region of IgG1.

DNA encoding VL is ligated to another DNA molecule encoding the light chain constant region CL, so as to obtain a full-length L chain gene (and a Fab light chain gene). The sequence of a human light chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The light chain constant region may be the constant region of κ or λ. The κ constant region may include any given various alleles known to be generated among different individuals, such as Inv (1), Inv (2) or Inv (3). The λ constant region may be derived from any one of the three λ genes.

The thus obtained H chain- or L chain-encoding DNA is inserted into a vector to produce an expression vector, and the produced expression vector is then allowed to express in a host cell. Thereafter, the secreted supernatant is recovered and purified to obtain a human antibody. Examples of the expression vector include a plasmid, retrovirus, adenovirus, adeno-associated virus (AAV), plant viruses such as cauliflower mosaic virus or tobacco mosaic virus, a cosmid, YAC, and EBV-derived episome. An expression vector and an expression regulatory sequence are selected, so that they are suitable for a host cell used for expression. An antibody light chain gene and an antibody heavy chain gene can be inserted into different vectors, or the two genes can also be inserted into a single expression vector. An antibody gene is inserted into an expression vector by a standard method (for example, ligation of a complementary restriction site on an antibody gene fragment to a vector, or blunt-ended ligation applied when no restriction sites are present).

A favorable vector encodes a functionally complete human CH or CL immunoglobulin sequence having a suitable restriction site, which has been produced by an engineering approach such that any given VH or VL sequence can be easily inserted and then expressed therein, as described above. In such a vector, splicing generally takes place between a splice donor site in the inserted J region and a splice acceptor site preceding a human C domain, or such splicing also takes place in a splice region existing in a human CH exon. Polyadenylation and transcription termination take place in a natural chromosomal site downstream of a coding region. A recombinant expression vector can also encode a signal peptide that promotes the secretion of an antibody chain derived from a host cell. An antibody chain gene can be cloned into a vector, such that a signal peptide can be ligated in-frame to the amino terminus of an immunoglobulin chain. The signal peptide may be either an immunoglobulin signal peptide or a heterogeneous signal peptide (namely, it may be a non-immunoglobulin protein-derived signal peptide).

An expression vector for the antibody may also have sequences such as a sequence for regulating replication of the vector in a host cell (e.g. a replication origin) or a selective marker gene sequence, as well as an antibody gene and a regulatory sequence. The selective marker gene promotes selection of a host cell into which a vector has been introduced. For instance, the selective marker generally imparts resistance to drugs such as G418, hygromycin or methotrexate to a host cell into which the vector has been introduced. Preferred selective marker genes include a dihydrofolate reductase (DHFR) gene (used in selection/amplification of methotrexate as a dhfr-host cell), a neomycin phosphotransferase gene (used in selection of G418), and a glutamate synthase gene.

A host cell is transformed with an antibody gene expression vector produced by the above-described method. Any type of cell may be used as a host cell, as long as it can produce the present antibody. Examples of such a host cell include bacteria, yeast, animal cells, insect cells, and plant cells. Among these cells, animal cells are preferable. Examples of the animal cells include Chinese hamster ovary cells CHO/dhfr(-) and CHO/DG44, monkey-derived cells COS (A. Wright & S. L. Morrison, J. Immunol. 160, 3393-3402 (1998)), and SP2/O cells (mouse myeloma) (K. Motmans et al., Eur. J. Cancer Prev. 5, 512-5199 (1996), R. P. Junghans et al., Cancer Res. 50, 1495-1502 (1990)). For transformation, a lipofectin method (R. W. Malone et al., Proc. Natl. Acad. Sci. USA 86, 6007 (1989), P. L. Felgner et al., Proc. Natl. Acad. Sci. USA84, 7413 (1987)), an electroporation method, a calcium phosphate method (F. L. Graham & A. J. van der Eb, Virology 52, 456-467 (1973)), a DEAE-Dextran method, and the like are preferably applied.

A transformant is cultured, and a human antibody is then separated from the cells of the transformant or a culture medium thereof. For separation/purification of the antibody, methods such as centrifugation, ammonium sulfate fractionation, salting-out, ultrafiltration, affinity chromatography, ion exchange chromatography and gel filtration chromatography can be used by appropriately combining them.

### Antibody Fragments

An antibody fragment can be produced based on the present antibody, or based on the sequence information of a gene encoding the present antibody. Examples of the antibody fragment include Fab, Fab', F(ab')₂, scFv, and dsFv antibodies.

Fab is obtained by digesting IgG by papain in the presence of cysteine. It is an antibody fragment with a molecular weight of approximately 50,000, which is constituted with L chain and H chain variable regions, and an H chain fragment consisting of a CH1 domain and a portion of a hinge region. In the present invention, the above-described antibody can be obtained by papain digestion. In addition, Fab can also be prepared by incorporating DNA encoding a portion of the H chain and the L chain of the above-described antibody into a suitable vector, then performing transformation with the resulting vector, and then obtaining it from the transformant .

Fab' is an antibody fragment with a molecular weight of approximately 50,000, which is obtained by cleaving a disulfide bond between the H chains of the below-mentioned F(ab')₂. In the present invention, Fab' can be obtained by digesting the above-described antibody by pepsin, and then cleaving a disulfide bond with a reducing agent. In addition, as with Fab, Fab' can also be prepared by genetic engineering using DNA encoding the Fab'.

F(ab')₂ is an antibody fragment with a molecular weight of approximately 100,000, which is obtained by binding, via a disulfide bond, one fragment (Fab') constituted with L chain and H chain variable regions and an H chain fragment consisting of a CH1 domain and a portion of a hinge region, to the other fragment (Fab'). In the present invention, F(ab')₂ can be obtained by digesting the above-described antibody by pepsin. In addition, as with Fab, F(ab')₂ can also be prepared by genetic engineering using DNA encoding the F(ab')₂.

scFv is an antibody fragment obtained by ligating the C-terminus of one chain of Fv consisting of an H chain variable region and an L chain variable region to the N-terminus of the other chain thereof, using a suitable peptide linker, so as to form a single chain. (GGGGS)₃ having high flexibility can be used, for example, as such a peptide linker. For instance, DNA encoding the H chain variable region and L chain variable region of the above-described antibody and DNA encoding a peptide linker are used to construct DNA encoding a scFv antibody, and the thus constructed DNA is then incorporated into a suitable vector. Thereafter, scFv can be prepared from a transformant obtained by transformation with the aforementioned vector.

dsFv is an Fv fragment obtained by introducing a Cys residue into a suitable site in each of an H chain variable region and an L chain variable region, and then stabilizing the H chain variable region and the L chain variable region by a disulfide bond. The site in each chain, into which the Cys residue is to be introduced, can be determined based on a conformation predicted by molecular modeling. In the present invention, for example, a conformation is predicted from the amino acid sequences of the H chain variable region and L chain variable region of the above-described antibody, and DNA encoding each of the H chain variable region and the L chain variable region, into which a mutation has been introduced based on such prediction, is then constructed. The thus constructed DNA is incorporated into a suitable vector. Thereafter, dsFv can be then prepared from a transformant obtained by transformation with the aforementioned vector.

Besides, it is also possible to ligate the scFv antibody to the dcFv antibody or the like using a suitable linker, or to fuse such an antibody fragment with streptavidin, so as to multimerize the antibody fragment.

### Multi-specific Antibody

The antibody may be a multi-specific antibody that recognizes TfR and simultaneously recognizes other targets. In particular, a bispecific antibody is often used.

The bispecific antibody means an antibody in which a molecule that recognizes TfR is combined with a molecule targeting another molecule.

Examples of the bispecific antibody may include bispecific (mab)₂ obtained by chemically crosslinking two molecules of monoclonal antibodies to each other, bispecific F(ab')2 obtained by chemically crosslinking two molecules of Fab fragments to each other, quadroma, bsDb (a bispecific diabody), scBsDb (a single-chain bispecific diabody), scBsTaFv (a single-chain bispecific tandem variable domain), Bite (a bispecific T cell Engager antibody), and DNL-F(ab)3 (docl-and-lock trivalent Fab) (Shim, H. Bispecific Antibodies and Antibody-Drug Conjugates for Cancer Therapy: Technological Considerations. Biomolecules 2020, 10, 360), but the forms of the bispecific antibody are not limited thereto.

### NK cell tumors

NK cell tumors include three types: aggressive NK-cell leukemia (ANKL), extranodal NK/T-cell lymphoma, nasal type (ENKL), and chronic lymphoproliferative disorders of NK cells (CLPD-NK).

### Pharmaceutical Composition and Preparation

The therapeutic agent for NK-cell tumor of the present invention can be provided as a pharmaceutical composition or a preparation.

The therapeutic agent for NK-cell tumor of the present invention can be used to treat NK-cell tumor in a subject who is in need of treatment of NK-cell tumor.

The therapeutic agent for NK-cell tumor of the present invention may comprises a physiologically acceptable diluent or carrier, in addition to the substance that recognizes a transferrin receptor. Examples of a suitable carrier used herein may include a normal saline, a phosphate buffered saline, a phosphate buffered saline with glucose, and a buffered saline, but the examples of the suitable carrier are not limited thereto. Otherwise, the substance that recognizes a transferrin receptor is freeze-dried or is frozen, and when needed, the aforementioned buffered aqueous solution may be added thereto to reconstitute the substance that recognizes a transferrin receptor, and the thus reconstituted substance that recognizes a transferrin receptor may be then used. The dosage form may be oral administration using tablets, capsules, granules, powdery agents, syrups, etc., or parenteral administration using injections (subcutaneous, intravenous, intramuscular, and intraperitoneal injections, etc.), transdermal, transmucosal, nasal, and transpulmonary administrations, suppository, etc.

The applied dose of the therapeutic agent for NK-cell tumor of the present invention is different depending on symptoms, age, body weight, etc. In general, in the case of oral administration, the present agent is administered at a dose of approximately 0.001 mg to 1,000 mg per day per adult, in terms of the amount of a substance that recognizes a transferrin receptor. Such a dose can be administered once or divided over several administrations per day. On the other hand, in the case of parenteral administration, the present agent can be administered at a dose of approximately 0.001 mg to 1,000 mg for a single administration, via subcutaneous, intramuscular, or intravenous administration.

The present invention will be described in more detail in the following examples. However, these examples are not intended to limit the scope of the present invention.

### Examples

In the following examples, the TfR436 antibody described in paragraphs 0090 and 0091 of International Publication WO2014/073641 was used.

The CDR sequences of the TfR436 antibody are shown below.
VH CDR1: SYGMH (SEQ ID NO: 1)
VH CDR2: VISYDGSNKYYADSVKG (SEQ ID NO: 2)
VH CDR3: DSNFWSGYYSPVDV (SEQ ID NO: 3)
VL CDR1: TRSSGSIASNSVQ (SEQ ID NO: 4)
VL CDR2: YEDTQRPS (SEQ ID NO: 5)
VL CDR3: QSYDSAYHWV (SEQ ID NO: 6)

The VH sequence and VL sequence of the TfR436 antibody are shown below.
TfR436 VH (SEQ ID NO: 7) 
DVQLVQSGGGVVQPGRSLRLSCAASGFPFKSYGMHWVRQAPGKGLEWVAVISYDGSNK
YYADSVKGRFTISRDNSKNTLYLQMNSLRGEDTAVYYCARDSNFWSGYYSPVDVWGQG
TTVTVSS
TfR436 VL (SEQ ID NO: 8)

NFMLTQPHSVSESPGKTVTISCTRSSGSIASNSVQWYQQRPGSAPITVIYEDTQRPSGVPDR
FSGSIDSSSNSASLTISGLQTEDEADYYCQSYDSAYHWVFGGGTKLAVL

### Example 1: Identification of binding site of TfR436 antibody

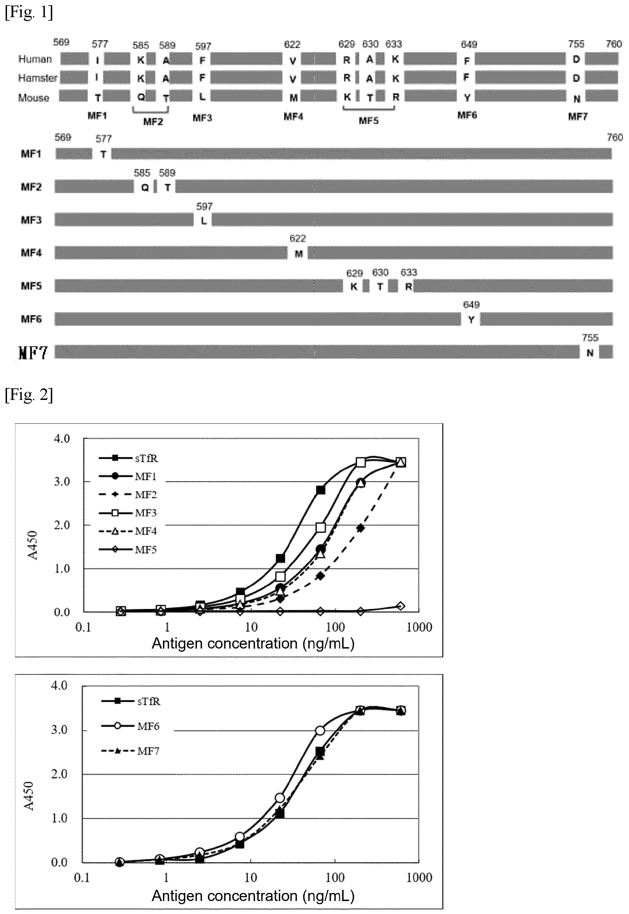

The TfR436 antibody did not cross-react with mouse TfR, but it exhibited cross-reactivity with hamster TfR. The amino acid sequence of a transferrin (TF)-binding site (the amino acids at positions 569 to 760) in TfR was aligned. The amino acids of a human TfR sequence, which were the same as hamster but were different from mouse, were picked up. The picked amino acids were subjected to point mutation, as shown in Fig. 1, so that soluble TfR mutant fragments were produced.

### (1) Production of soluble wild-type TfR (sTfR) and TfR mutant fragments (MF1 to MF7)

A human TfR extracellular domain (the amino acids at positions 89 to 760) or each TfR mutant fragment (MF1 to MF7) as shown in Fig. 1, and a nucleotide sequence encoding AAARGGPEQKLISEEDLNSAVDHHHHHH (SEQ ID NO: 10) were totally synthesized. A neomycin resistance gene and a DHFR gene were incorporated into the expression vector pCAGGS (Non-Patent Document 2.: Niwa et al. 1991), and thereafter, the synthesized genes were each inserted into the multicloning site of the obtained expression vector, so as to produce a pCAGGS-Neo-DHFR-sTFR-myc-his expression plasmid. Using Expifectamine (Thermo Fisher Scientific), the Expi293 cells (Thermo Fisher Scientific) were transfected with the above-described plasmid, and were then cultured at 37°C in 8% CO₂ at 135 rpm for 5 days. Thereafter, a culture supernatant was recovered by centrifugation. A HisTrapHP (Cytiva) column was connected with AKTA prime (Cytiva), and sTfR or MF1 to MF7 were then purified using 20 mM Imidazole/DPBS as a binding buffer and 500 mM Imidazole/DPBS as an elution buffer. The eluted protein was subjected to buffer exchange with 30 mM HEPES, 5% trehalose, pH 7.2, by using a Zeba spin column (Thermo Fisher Scientific).

### (2) Identification of binding site of TfR436 antibody

The thus purified sTfR or MF1 to MF7 were diluted with PBST (Phosphate Buffered Saline with Tween 20, TaKaRa), and the diluted solutions were prepared at 7 stages by 3-fold dilution from 600 ng/mL. Thereafter, the diluted solution was dispensed into Ni-NTAHisSorb Strips 96-well plate (QIAGEN) in an amount of 100 µL/well, and the plate was then placed on a shaker, followed by performing a reaction at room temperature. One hour later, the reaction mixture was washed with PBST Buffer 5 times, and the TfR436 antibody (1 ug/mL) was dispensed into the 96-well plate in an amount of 100 µL/well. The plate was placed on a shaker, and a reaction was then performed at room temperature for 1 hour. Thereafter, the reaction mixture was washed with PBS-T Buffer 5 times, and a 50,000-fold diluted secondary antibody, F(ab')2 Fragment Anti-Human IgG Fcγ (Jackson Immuno Research), was then dispensed into the 96-well plate in an amount of 100 µL/well, followed by performing a reaction at room temperature for 1 hour. The reaction mixture was washed with PBST Buffer 5 times, and TMB Soluble Reagent (High Sensitivity) (Scy Tek) was then dispensed into the plate in an amount of 100 µL/well, followed by performing a reaction at room temperature in a dark place for 3 minutes. After that, TMB Stop Buffer (Scy Tek) was added to the plate in an amount of 100 µL/well, and the obtained mixture was then shaken using a shaker for 1 minute. Thereafter, the absorbance at 450 nm (ref. 620 nm) was measured using a plate reader.

As a result, as shown in Fig. 2, a reduction in the reactivity of the TfR436 antibody with the TfR mutant fragment MF5 was observed, but a reduction in the reactivity of the TfR436 antibody with other mutant fragments was not observed. That is to say, when the amino acids at positions 629, 630 and 633 of TfR are substituted with other amino acids, the TfR436 antibody cannot recognize the TfR. Thus, it was suggested that the amino acids at positions 629 to 633 of TfR should be an epitope recognized by the TfR436 antibody.

### Example 2: Comparison between TfR436 antibody and comparative antibodies in terms of Tf-TfR binding inhibition

### (1) Production of comparative antibody A24

Patent Document US2008/0193453 discloses an A24 antibody reacting against human TfR. In order to compare the TfR436 antibody with the A24 antibody, the deposited hybridomas were acquired, and the antibody was produced. Specifically, the hybridomas were seeded into RPMI1640 (GIBCO) supplemented with 10% FBS to a cell concentration of 1 to 2 × 10⁵/mL, and were then cultured in a 5% CO₂ incubator at 37°C. After completion of an expansion culture, the cells were recovered by centrifugation, and were then washed with PBS twice. Thereafter, the resulting cells were further subjected to an expansion culture in a serum-free medium Cosmedium 005 (Cosmo Bio Co., Ltd.) and 0.5% Nutridoma-CS (Roche) to result in a volume of 550 mL. Five days after the cells became confluent, a culture supernatant was recovered by centrifugation.

The recovered supernatant was applied onto a protein A carrier (Ab-Capcher ExTra: ProteNova), and an antibody binding to protein A was eluted with a 0.1 M glycine-HCl buffer (pH 2.7), and then, was rapidly neutralized with a 1 M Tris-HCl buffer (pH 8.5). Thereafter, using an Ultracell Ultrafiltration Disk (Merck), the buffer was exchanged with PBS.

### (2) Comparison between TfR436 antibody and antibody of other company in terms of Tf-TfR binding inhibition

The sTfR described in Example 1 was adjusted to a concentration of 5.0 µg/mL by addition of PBST, and the diluted solution was then dispensed into MaxiSorp 96-well plate (Nunc) in an amount of 100 µL/well. Thereafter, it was left at rest at 4°C overnight to obtain a solid phase. On the following day, the solid phase solution was discarded, and 100% Block Ace (DS Pharma Biomedical) was added to the plate in an amount of 200 µL/well, followed by leaving the obtained mixture at rest at room temperature so as to carry out blocking. One hour later, the reaction mixture was washed with PBST Buffer 5 times, and HRP-labeled Tf (2 ug/mL) was then dispensed into the plate in an amount of 50 µL/well. Thereafter, the TfR436 antibody, the A24 antibody (2-fold dilution series from 10 µg/mL), or holo-Tf (Merck) (2-fold dilution series from 300 µg/mL) was further added to the mixture in an amount of 50 µL/well. The obtained mixture was reacted at room temperature for 1 hour, and was then washed with PBST Buffer 5 times. Thereafter, TMB Soluble Reagent (High Sensitivity) was dispensed into the plate in an amount of 100 µL/well, and the obtained mixture was then reacted at room temperature in a dark place. Twenty five minutes later, TMB Stop Buffer was added to the plate in an amount of 100 µL/well, the obtained mixture was then shaken with a shaker for 1 minute, and the absorbance at 450 nm (ref. 620 nm) was then measured using a plate reader.

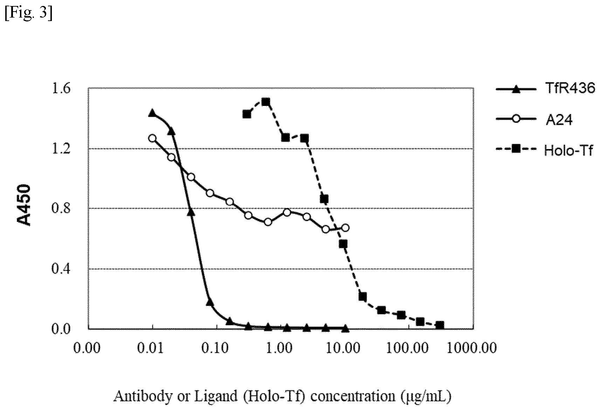

As a result, as shown in Fig. 3, the TfR436 antibody completely inhibited the binding of Tf-TfR at a significantly low dose (100 ng/mL). In contrast, the A24 antibody could not completely inhibit the binding of Tf-TfR, even though it was used at a dose of 10 µg/mL, and could inhibit only 50% of the Tf-TfR binding. Thus, it was suggested that the TfR436 antibody should be excellent in terms of inhibition of the Tf-TfR binding.

### Example 3: Cytotoxicity test (in vitro)

As cells, KHYG-1, NK92, SNK1, SNK10, and SNK6 were used.

KHYG-1 and NK92 are cell lines established from ANKL cells, whereas SNK1, SNK10, and SNK6 are cell lines established from ENKL cells. ENKL is extranodal NK/T-cell lymphoma, nasal type (ENKL), that is an NK-cell tumor, as with ANKL.

An appropriate number of cells (3 × 10⁴ cells for the cell lines, and 5 × 10⁵ cells for patient-derived cells) were seeded into 18 wells of a 24-well plate, using 800 µL of Artemis-Medium 1 + 2% human serum.

TfR436 antibody was added into the culture medium to each concentration (10,000 ng/mL, 1,000 ng/mL, 100 ng/mL, 10 ng/mL, 1 ng/mL, and 0 ng/mL).

The cells were cultured at 37°C for 48 hours (under 5% CO₂ conditions).

The cells were recovered by centrifugation for each well, and were then stained with an anti-human CD45-APC antibody and an anti-human CD56-PE antibody (Biolegend) that had been diluted with FACS buffer (1% FBS, Funakoshi Co., Ltd.; PBS, Funakoshi Co., Ltd.) to the designated concentrations (see attached documents for each reagent) for 15 minutes (the antibody staining is not necessary for the cell lines).

After washing with FACS buffer, the cell suspension was adjusted again with 500 µL/well of FACS buffer.

The number of events of APC-positive and PE-positive cell population was counted by Flow cytometry (Becton Dickenson) at a low speed for 30 seconds fixed per well, and was recorded as the number of viable cells.

Using 0 ng/mL as a control, the ratio of the number of viable cells at each concentration was calculated and was then plotted.

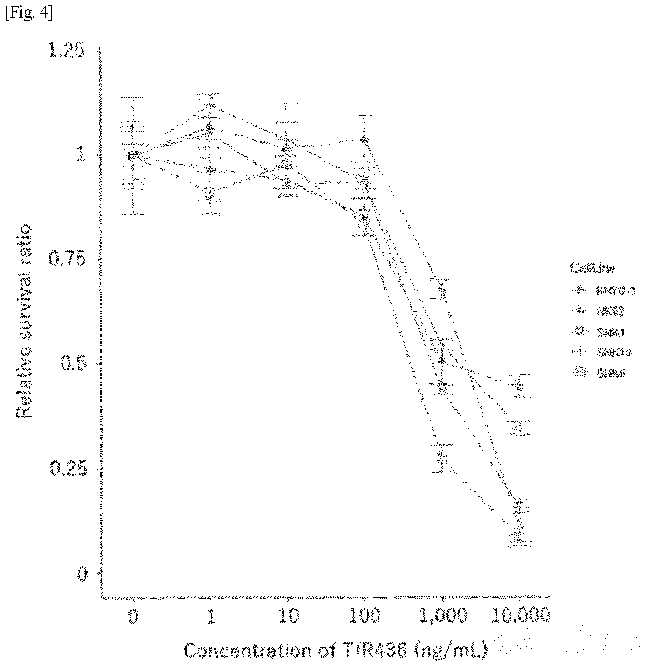

The results are shown in Fig. 4.

### Example 4:

The IVIS and histology of mice with and without antibody administration are shown.

Patient-derived ANKL cells (1 × 10⁶ cells per mouse), into which a firefly luciferase gene had been introduced by lentivirus, were administered via the tail vein into 7 to 11-week-old female NOG mice.

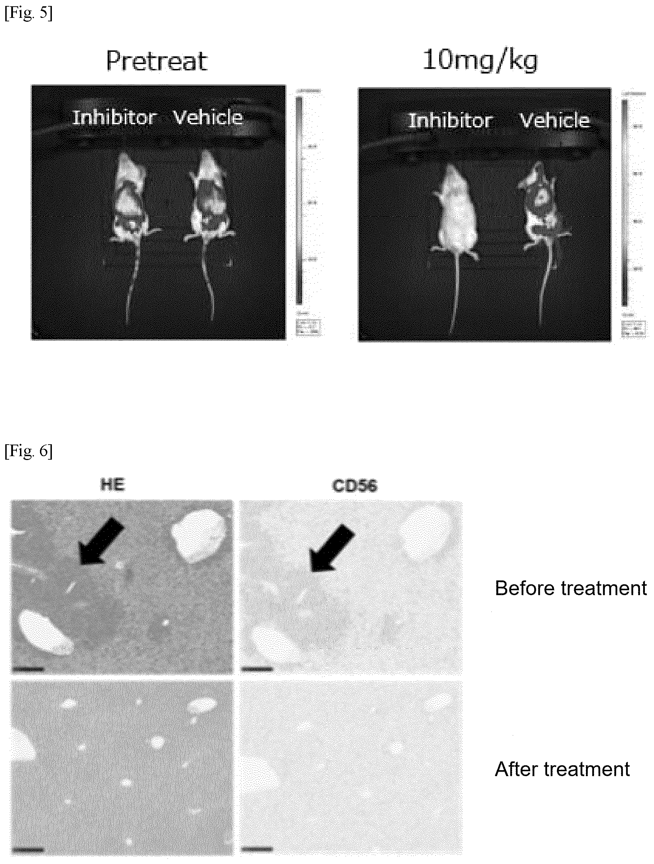

On Days 5, 7, 14, 18, and 25 after the administration, 150 mg/kg luciferin was intraperitoneally administered into the mice, into which systemic anesthesia had been introduced by inhalation of isoflurane, and imaging was performed by IVIS with normal sensitivity and 1-minute exposure over time starting 10 minutes after the administration. The TfR436 antibody was administered on the 6th day after the administration. The results are shown in Fig. 5.

Liver and spleen were fixed in 4% formalin, and thin sections were then prepared therefrom, and thereafter, EBER *in situ* hybridization (Bond ISH EBER; Probe, #PB0589) was performed.

The results are shown in Fig. 6.

### Example 5:

For each of 7 to 11-week-old female NOG mice, 1 × 10⁶ patient-derived ANKL cells were administered.

On Days 5, 8, 12, and 15 after the administration, 20 mg/kg TfR436 antibody (the total amount of which was increased to 200 µL with PBS) or 200 µL of PBS was administered to the mice.

The humane endpoints were defined as a weight loss of 15% or more from the peak body weight, significant weakness (a level of difficulty in walking on their own) or death, and the number of days to the endpoint was analyzed by the Kaplan-Meier method.

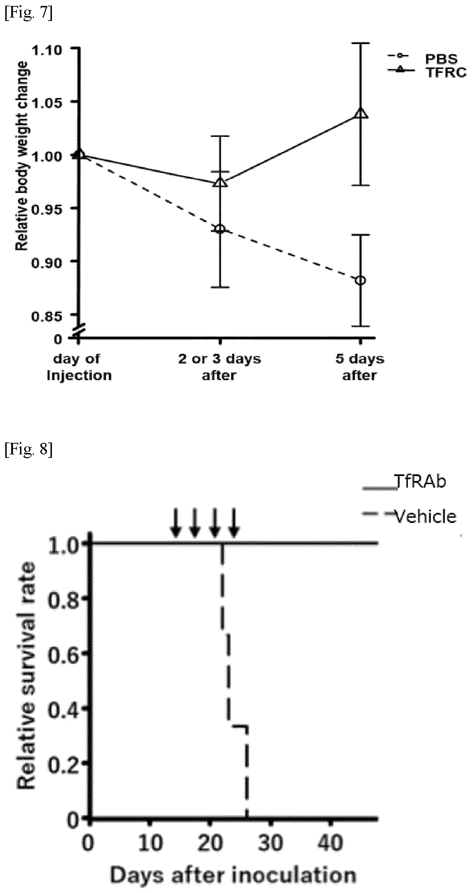

Fig. 7 shows a transition in the body weight of the mice. Fig. 8 shows the results of the survival test of mice.

### Example 6: In vivo competitive assay using CRISPR-Cas9 system

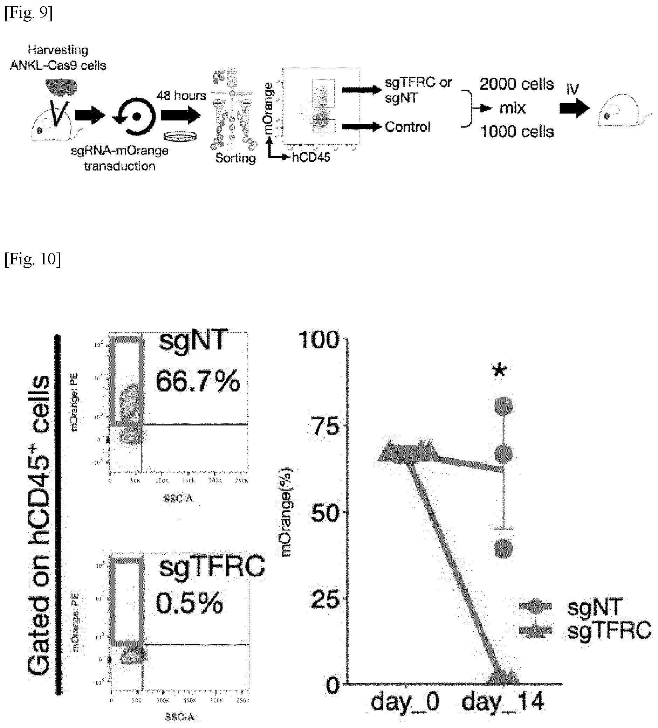

Fig. 9 shows an *in vivo* competition assay method using a CRISPR-Cas9 system. Patient-derived ANKL cells were administered to female NOD/Scid, IL2-Rgnull (NOG) mice via the tail vein, and 3 to 4 weeks after the administration, the ANKL cells are harvested from the liver (hereafter, the mice administered with patient-derived cells (ANKL1) are referred to as "PDX mice"). These ANKL cells were transfected with an FUGW-Cas9-Venus vector (YFP (Venus) was inserted into lentiCas9-Blast (Addgene, a non-profit plasmid bank) for modification). The obtained cells were defined as ANKL1-Cas9 cells and were then administered to new NOG mice via the tail vein. Three to four weeks later, the cells were recovered from the livers of established PDX mice. Thereafter, the recovered cells were sorted using a cell sorter based on Venus expression, and were then administered again to NOG mice. This recovery-sorting-administration process was performed twice to obtain pure Venus+ANKL (ANKL-Cas9) cells. The ANKL-Cas9 cells were cryopreserved for use in the subsequent experiments. Thereafter, a CSII-sgRNA-mOrange vector (CSII vector: RIKEN #RDB04378, into which the lentivirus packaging gene described in Mol Ther. 2002 Mar; 5(3): 242-51. doi: 10.1006/mthe.2002.0549. PMID: 11863413was cloned) was used, and a single guide RNA targeting a transferrin receptor antibody gene (sgTFRC) and a single guide RNA targeting a sequence that was not present on the human gene sequence (sgNT) were introduced into ANKL-Cas9 cells. Forty-eight hours later, hCD45+mOrange+ cells (ANKL cells in which the target gene was knocked out by the single guide RNA) and hCD45+mOrange -cells (ANKL cells nearly in a parental state without introduction of the single guide RNA) were sorted using a cell sorter, and the sorted cells were then mixed at a ratio of 2000 cells to 1000 cells. The thus mixed cells were transplanted into mice. After 3 to 4 weeks after the transplantation, ANKL cells were harvested from the liver, and were then analyzed in terms of the abundance ratio of the hCD45+mOrange+ cells, so as to analyze the effects of the knocking out of the target gene on viability in the liver.

Fig. 10 shows a comparison of sgNT- or sgTFRC-introduced ANKL1-Cas9 cells in the liver. The sgNT- or sgTFRC-introduced ANKL1-Cas9 cells, which had been established by the method shown in Fig. 9, were transplanted into NOG mice, and on Day 0 and Day 14, the percentage of mOrange+ cells in the mouse liver (n = 3) was analyzed using flow cytometry.

As shown in Fig. 10, the sgTFRC-introduced ANKL1-Cas9 cells were confirmed to be deficient in TFRC. From the results shown in Fig. 10, it was demonstrated that the knocking-out of TFRC in aggressive NK cells can suppress proliferation of the aggressive NK cells (i.e., the aggressive NK cells cannot survive).

### Example 7: Viability of ANKL cell lines treated with various concentrations of TfR436 antibody

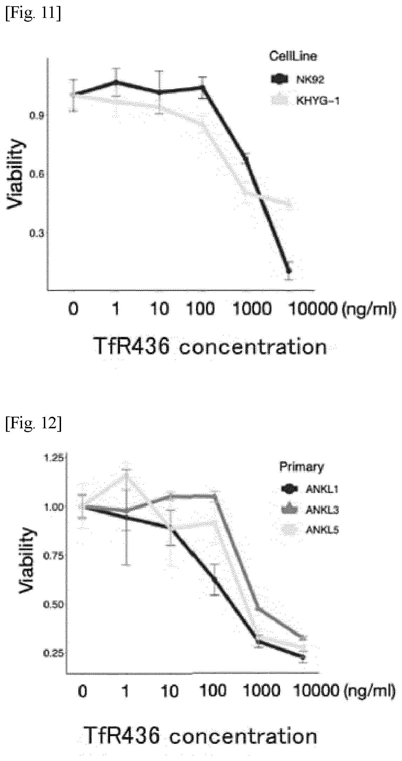

Fig. 11 shows the viability of the ANKL cell lines treated with various concentrations of TfR436 antibody. 1000 cells per well of the NK leukemia cell lines (NK92 and KHYG-1) were treated with various concentrations of TfR436 antibody for 96 hours, and thereafter, the viability of each cell line after the treatment was evaluated using the MTT cell counting kit (Nacalai Tesque, #23506-80).

With regard to the IC50 of each cell line, it was 1155 ng/mL in the case of NK92, and it was 242 ng/mL in the case of KHYG-1.

### Example 8: Viability of primary ANKL cells treated with various concentrations of TfR436 antibody

Fig. 12 shows the viability of the primary ANKL cells treated with various concentrations of TfR436 antibody. The cells of individual ANKL patients (ANKL1, ANKL3, and ANKL5) were transplanted into mice by intravenous tail injection, and 3 to 4 weeks after the transplantation, ANKL cells were obtained from the liver of the PDX mice. Thereafter, 1000 cells per well of the obtained ANKL cells were seeded into the wells, and the cells were then treated with various concentrations of TfR436 antibody for 48 hours. Thereafter, the *in vitro* viability of the cells was evaluated using MTT cell counting kit (Nacalai Tesque, #23506-80).

With regard to the IC50 of each patient-derived cells, it was117 ng/mL in the case of ANKL1; it was 754 ng/mL in the case of ANKL3; and it was 262 ng/mL in the case of ANKL5.

### Example 9: Verification of in vivo efficacy of TfR436 antibody using ANKL-PDX mice

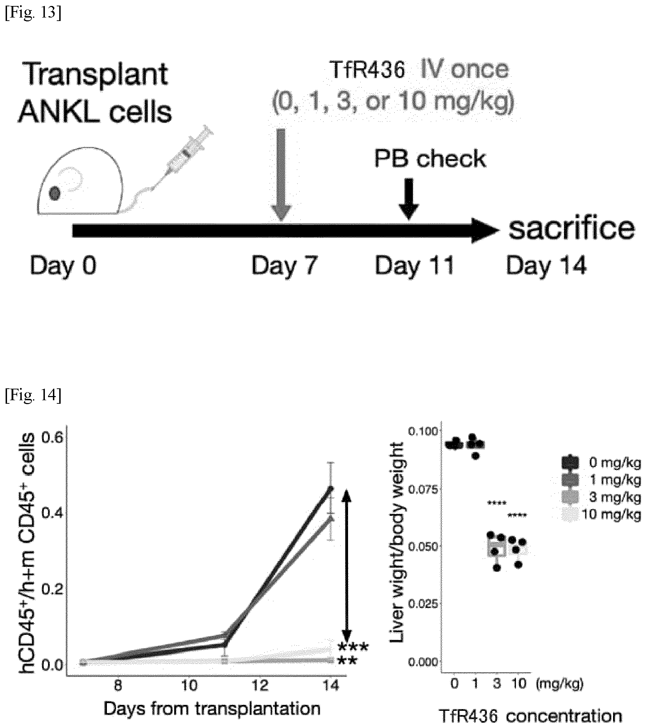

Fig. 13 shows the procedures for validation of the efficacy of a TfR436 antibody *in vivo* using ANKL-PDX mice. ANKL1 cells were intravenously transplanted into NOG mice, and 7 days after the transplantation, a TfR436 antibody was administered at doses of 0, 1, 3, and 10 mg/kg. Blood was collected 11 days later. The mice were sacrificed on the 14th day, and ANKL cells were then harvested from the liver.

Fig. 14 shows the ratio of human CD45+ cells to mouse CD45+ cells. The ratio of the human CD45+ cells to the mouse CD45+ cells in the peripheral blood of ANKL1-PDX mice that had been administered with 0, 1, 3, and 10 mg/kg of the TfR436 antibody, on Days 11 and 14 after the transplantation, was analyzed using a flow cytometer (left). Liver weights per body weight of the ANKL 1-PDX mice (n = 4) were measured among 0, 1, 3 and 10 mg/kg administrations of the TfR436 antibody (right).

As a result, proliferation of the ANKL cells in the livers of the PDX mice was suppressed by administration of the TfR436 antibody.

### Example 10: RNA-seq analysis of ANKL1-PDX mice

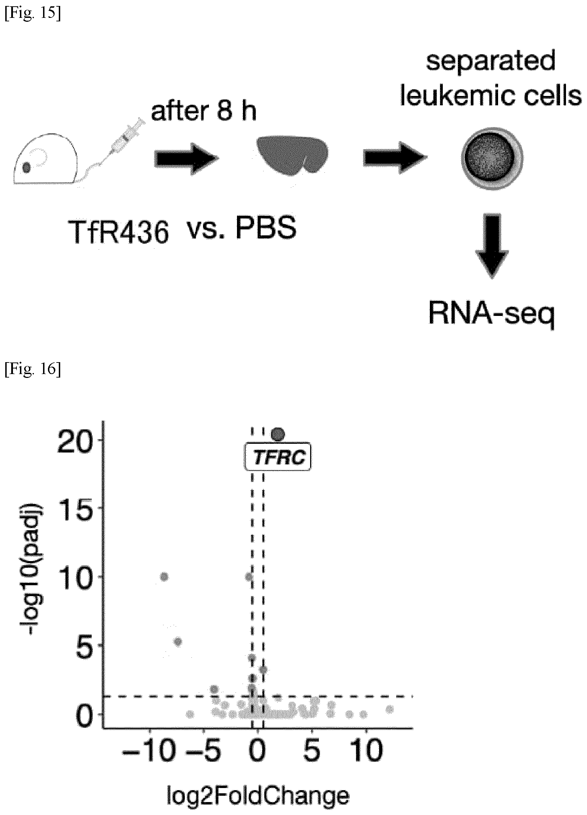

Fig. 15 shows the method of RNA-seq analysis of ANKL1-PDX mice. ANKL1 cells (1 × 10⁶ cells) were injected into NOG mice by tail vein injection to establish PDX mice, and 14 days after administration of the ANKL1 cells, a TfR436 antibody (10 mg/kg) or PBS was administered to the PDX mice by tail vein injection. Eight hours after the administration, ANKL cells were harvested from the liver, and were then subjected to RNA-seq analysis (n = 3).

Fig. 16 shows a volcano plot of the RNA-seq data. The expression level ratio was plotted on the horizontal axis, and -log10 (adjusted p-value) was plotted on the vertical axis. As a result, TFRC showed a prominent change.

### Example 11: Measurement of iron amount in ANKL3 cells

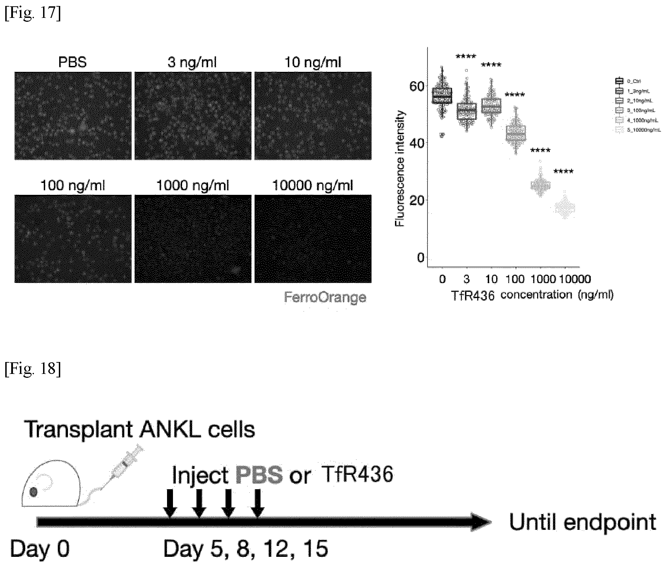

Fig. 17 shows the results of the measurement of iron amount in ANKL3 cells.

(Methods) ANKL3 cells were treated *in vitro* with 0, 1, 10, 100, 1000, and 10000 ng/mL of TfR436 antibody, and 48 hours after the treatment, the cells were stained with an intracellular iron ion measurement reagent (FerroOrange (registered trademark); Dojindo, #342- 09533). The intracellular Fe²⁺ concentration was measured by quantifying the fluorescence intensity under a confocal microscope. *: p < 0.05; **: p < 0.01; ***: p < 0.001; and ****: p < 0.0001.

The intracellular Fe²⁺ amount was decreased depending on the amount of the TfR436 antibody used in the treatment.

### Example 12: In vivo evaluation of therapeutic effects of TfR436 antibody

Fig. 18 shows the procedures for *in vivo* evaluation of the therapeutic effects of TfR436 antibody. ANKL cells (1 × 10⁶ cells) were transplanted into ANKL-PDX mice, and on Days 5, 8, 12, and 15 after the transplantation, PBS or 10 mg/kg TfR436 antibody was administered to the mice. Thereafter, the mice were monitored until the endpoint.

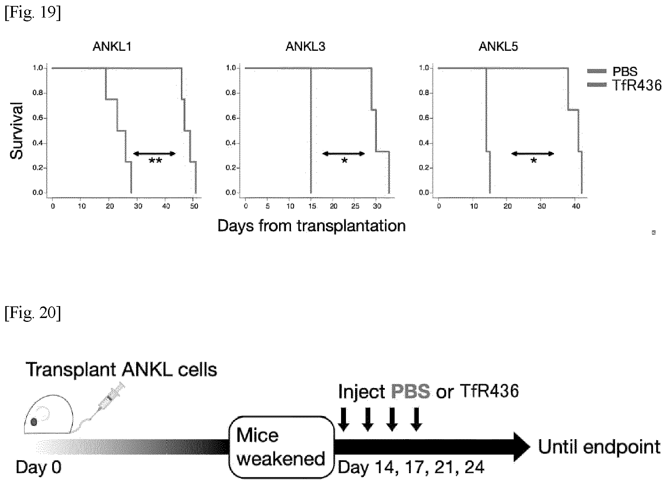

Fig. 19 shows the survival curves of ANKL1, ANKL3, and ANKL5 PDX mice. For each of 7 to 11-week-old female NOG mice, 1 × 10⁶ patient-derived ANKL cells were administered. On Days 5, 8, 12, and 15 after the administration, 10 mg/kg TfR436 antibody (the total amount of which was increased to 200 µL with PBS) or 200 µL of PBS was administered to the mice.

The humane endpoints were defined as a weight loss of 15% or more from the peak body weight, significant weakness (a level of difficulty in walking on their own) or death, and the number of days to the endpoint was analyzed by the Kaplan-Meier method (n = 3 to 4 in each group). The body weight was corrected with the body weight on Day 14.

The survival period was prolonged in all ANKL cells.

### Example 13: Efficacy of treatment with TfR436 antibody in ANKL-PDX mice with advanced symptoms

Fig. 20 shows the procedures for evaluating the efficacy of a treatment with a TfR436 antibody in ANKL-PDX mice with advanced symptoms. ANKL1 cells were transplanted into ANKL-PDX mice, and at a time point when the mice were weakened, PBS or a TfR436 antibody were administered to the mice. Then, the mice were monitored until the endpoint.

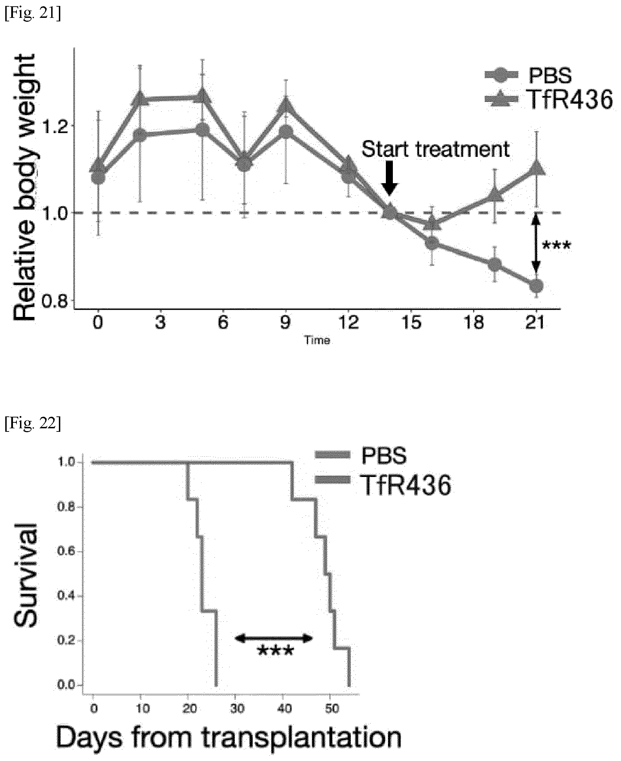

Fig. 21 and Fig. 22 show the therapeutic effects of a TfR436 antibody in ANKL1-PDX mice with advanced symptoms. For each of 7 to 11-week-old female NOG mice, 1 × 10⁶ patient-derived ANKL cells were administered. On Days 14, 17, 21, and 24 after the administration, 10 mg/kg TfR436 antibody (the total amount of which was increased to 200 µL with PBS) or 200 µL of PBS was administered to the PDX mice.

The humane endpoints were defined as a weight loss of 15% or more from the peak body weight, significant weakness (a level of difficulty in walking on their own) or death, and the number of days to the endpoint was analyzed by the Kaplan-Meier method (n = 6). The body weight was corrected with the body weight on Day 14.

Even PDX mice with advanced symptoms, the survival period thereof was prolonged by administration of the TfR436 antibody, and the TfR436 antibody exhibited therapeutic effects.

### Example 14: IVIS analysis of ANKL1-PDX mice treated with TfR436 antibody or PBS

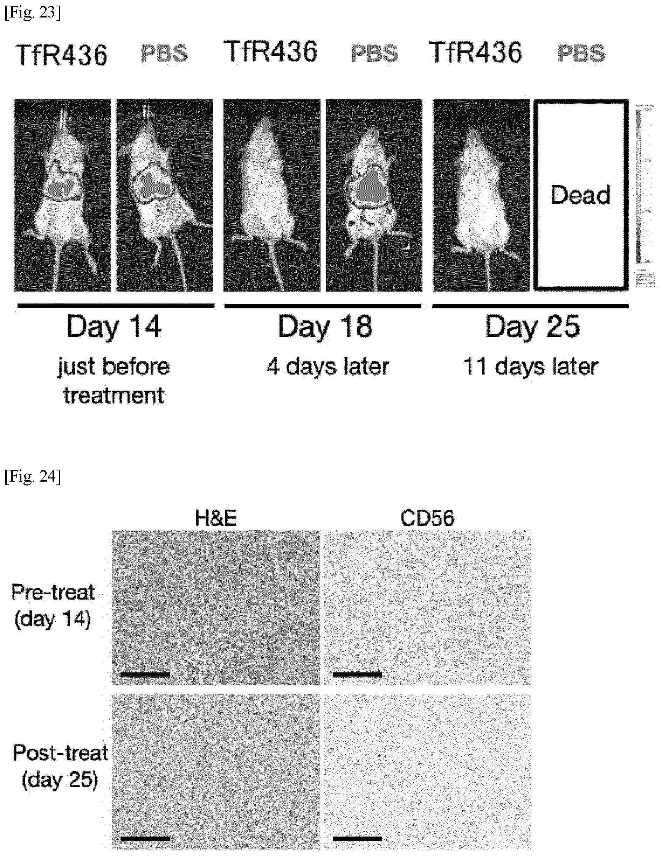

Fig. 23 shows the IVIS analysis of ANKL1-PDX mice treated with a TfR436 antibody or PBS. Patient-derived ANKL cells (1 × 10⁶ cells per mouse), into which a firefly luciferase gene had been introduced by lentivirus, were administered via the tail vein into 7 to 11-week-old female NOG mice, thereby establishing PDX mice. On Days 14, 17, 21, and 24 after the administration of the ANKL cells, a TfR436 antibody (10 mg/kg) or PBS was administered to the mice via the tail vein.

On Days 14, 17, 21, and 24 after the administration (in all cases, just before the time of administration of the TfR436 antibody or PBS on each day), 150 mg/kg luciferin was intraperitoneally administered into the mice, into which systemic anesthesia had been introduced by inhalation of isoflurane, and imaging was performed by IVIS with normal sensitivity and 1-minute exposure over time starting 10 minutes after the administration.

The ANKL cells disappeared by administration of the TfR436 antibody.

### Example 15: Therapeutic effects of TfR436 antibody

Fig. 24 shows the results of immunostaining of livers from mice administered with a TfR436 antibody. ANKL1 and ANKL3 (1 × 10⁶ cells each) were each administered to two different NOG mice, so as to establish PDX mice. On Day 14 after the establishment of the PDX mice, the liver was removed from one mouse of each group. To the remaining one of each group, 10 mg/kg TfR436 antibody was administered via the tail vein on Days 14, 17, 21, and 24 after the establishment of the mice, and on Day 25, the liver was removed from each mouse. The removed liver was immediately fixed with 4% formalin, and thin sections were then prepared. Thereafter, Hematoxylin-Eosin staining and immunostaining using a clinical diagnostic standard anti-human CD56 antibody were carried out. Scale bar: 100 µm. *: p < 0.05; **: p < 0.01; ***: p < 0.001; and ****: p < 0.0001.

No ANKL cells were observed on Day 25 after the administration, and the TfR436 antibody exhibited therapeutic effects.

## Claims

1. A therapeutic agent for NK-cell tumor, which comprises a substance that recognizes a transferrin receptor.

2. The therapeutic agent according to claim 1, wherein the NK-cell tumor is aggressive NK-cell leukemia, or extranodal NK/T-cell lymphoma, nasal type (ENKL).

3. The therapeutic agent according to claim 1 or 2, wherein the transferrin receptor-recognizing substance is a transferrin receptor-recognizing antibody.

4. The therapeutic agent according to claim 3, wherein the transferrin receptor-recognizing antibody is a human transferrin receptor-recognizing antibody.

5. The therapeutic agent according to claim 4, wherein the human transferrin receptor-recognizing antibody is an antibody that recognizes the amino acids at positions 629 to 633 of a human transferrin receptor, or an antibody that inhibits the binding of another antibody to the amino acids at positions 629 to 633 of a human transferrin receptor.

6. The therapeutic agent according to claim 3, wherein the antibody is an antibody having a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and also having a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.

7. The therapeutic agent according to claim 3, wherein the antibody is an antibody having a heavy chain as set forth in SEQ ID NO: 7 and a light chain as set forth in SEQ ID NO: 8.

8. The therapeutic agent according to claim 3, wherein the antibody is a human antibody or a humanized antibody.

9. The therapeutic agent according to claim 3, wherein the antibody is an antibody fragment selected from the group consisting of Fab, Fab', F(ab')₂, a single-chain antibody (scFv), a multi-specific antibody, a disulfide-stabilized V region (dsFv) and a peptide comprising CDR.
